# EUROPEAN PATENT APPLICATION

(11) **EP 4 613 370 A1**
(43) Date of publication of application: **10.09.2025**
(21) Application number: 24161110.2
(22) Date of filing: 04.03.2024
(51) Int. Cl.: B01J 20/287, B01D 15/32, B01D 15/36, B01D 15/38, B01J 20/32

(54) **MIXED MODE CHROMATOGRAPHIC PACKING MATERIAL**

(71) Applicant: Dionex Softron GmbH, 82110 Germering (DE)
(72) Inventor: BAZIULYTE-PAULAVICIENE, Dovile, Vilnius (LT); JANSONS, Martins, Vilnius (LT); WILLIAMS, Richard, Hemel Hempstead (GB); DAMONSKIS, Matas, Vilnius (LT)
(74) Representative: Robinson, Louise Frances

(57) **Abstract**

The present invention relates to the field of chromatographic sample separation that includes liquid chromatography and solid phase extraction and, in particular, it relates to material and the synthesis of material for use as a stationary phase in chromatographic sample separation. The invention further relates to uses of the material, in particular in the separation of hydrophilic and hydrophobic peptides, non-glycosylated and N-linked glycosylated peptides, deamidated and oxidized peptides. The invention also relates to chromatographic columns and solid phase extraction columns containing the material as a stationary phase.

## Description

### Field of the Invention

The present invention relates to the field of chromatographic sample separation that includes liquid chromatography and solid phase extraction and, in particular, it relates to material and the synthesis of material for use as a stationary phase in chromatographic sample separation. The invention further relates to uses of the material, in particular in the separation of hydrophilic and hydrophobic peptides, non-glycosylated and N-linked glycosylated peptides, deamidated and oxidized peptides. The invention also relates to chromatographic columns and solid phase extraction columns containing the material as a stationary phase.

### Background of the Invention

Liquid chromatography (LC), e.g. HPLC and UHPLC, and solid phase extraction (SPE) are used routinely in both analytical and preparative chromatography applications for determination of the quality and quantity of analytes in a variety of samples. In these chromatographic techniques, separation of a sample comprising a mixture of components (also termed analytes) is achieved by conveying the sample in a liquid mobile phase through a stationary phase in a column, thereby causing the sample to separate into its components due to different partitioning between the mobile and stationary phases of each of the components (i.e. the components have different partition coefficients). The stationary phase is typically in the form of a bed of particles packed within the column, or in the form of a monolithic material held in the column. Silica particles are commonly used as the stationary phase, either as nonporous, fully porous or superficially porous particles. Silica based HPLC columns are used for a broad range of applications because of their excellent physical strength, high efficiency and mature surface bonding chemistry.

The selectivity of a stationary phase for analytes is mainly governed by the column chemistry, which is key in LC separation. The column chemistry is routinely controlled by modifying the surface of the stationary phase, commonly by bonding compounds to the surface. The introduction of various functional groups onto the stationary phase surface enables the implementation of mixed-mode chromatography, where multiple separation mechanisms can be employed simultaneously.

However, silica-based columns have pH limitations. Under acidic conditions, the bonded compounds can be cleaved at the siloxane (Si-O-Si) linkage between the silica surface and the compound, resulting in loss of hydrophobic retention in the case of a C₁₈ bonded column. Additionally, silica particles are susceptible to dissolution at low pH, leading to the loss of chromatographic performance and reduced column lifetime. Under alkaline conditions, the hydroxide ions can erode the silica substrate by destroying the siloxane linkages in the silica backbone, causing the collapse of the packed bed or headspace (void) in the column.

Stationary phase media for HPLC separations are commonly produced by modifying a silica surface with silylating agents. Monofunctional silylating agents are often used to form monolayer surface coatings, while di- and tri-functional silylating agents are used to form polymeric coatings on silica surfaces generally resulting in improved chemical stability (GB Pat. No. 2,549,417). However, the use of some silylating agents results in coatings with undesirable properties including instability to hydrolysis and inadequate masking of acidic silanols on silica surfaces.

The presence of acidic silanols on the surface of silica particles has been a known challenge in LC. This is because acidic silanols can cause irreversible adsorption of basic solutes, resulting in strong peak asymmetry and a significant dependence of retention time on solute concentration. To mitigate this issue, end-capping techniques using activated silanes such as hexamethyldisilazane or bis-trimethylsilylacetamide have been employed to improve the behaviour of bonded phases (US Pat. No. 5,134,110, US Pat. No.7,534,352). However, this approach is primarily applicable to RP materials and presents a limitation when introducing polar functional groups for different selectivity in mixed-mode stationary phases, as these groups can react with the end-capping silanes, compromising the stability and performance of the stationary phase.

C18 HPLC columns are predominantly used for in RP HPLC, where the primary interaction between the stationary phase and analyte is hydrophobic interactions, arising from dispersion forces.

Secondary interactions of C18 phases without end-capping involve hydrophilic interactions between residual silanol groups and analyte.

Conventional C18 columns are well known in the art and are designed to achieve separation based on specific requirements, altering product characteristics such as: 1) particle's pore size: smaller pore sizes (e.g.: 80Å) are suitable for smaller molecules, while bigger pore sizes (e.g.: 300Å) are more suitable for larger molecules; 2) particle's surface area: higher surface area leads to higher retention; 3) particle size: smaller particles (e.g.: 1.5µm) leads to higher separation efficiency, while bigger particles (e.g.: 5µm) results in lower column backpressure; 4) column dimensions: longer columns (e.g.: 20cm length) provides better separation and resolution, while shorter columns (e.g.: 5cm length) enables faster separation and analysis; 5) specific end-capping is employed to reduce silanol activity and increase particle stability.

Conventional C18 columns find extensive applications in various industries such as pharmaceuticals, environmental analysis, food analysis, and others. C18 phase is commonly used for the analysis of small molecules, peptides, proteins and other compounds of interest.

However, while conventional C18 columns are effective in separating non-polar and moderately polar compounds, they possess limited selectivity towards highly polar and ionic compounds and their sample mixtures. This limitation necessitates considering new paths towards enhanced separation and resolution of analytes of interest such as alternative chemistries, mixed-mode approaches, or implementation of multi-dimensional LC techniques. One of particular area is proteomics and peptide analysis where a combination of hydrophobic and ion-exchange interactions can be utilized for improved separation and resolution.

Hybrid materials can address some chromatographic challenges, including improved stability at both high and low pH levels (US Pat. No.9,120,083). However, when the testing environment changes, hybrid materials have a tendency to show a drift in retention time, leading to poor peak shape for bases at low pH, that negatively affects loadability and peak capacity. Moreover, the presence of surface organic groups, particularly methyl groups, leads to lower concentrations of the bonded phase on the surface, thereby impairing the efficiency of chromatographic separation.

Therefore, while end-capping technique and hybrid materials have proven to be beneficial for improving the characteristics of LC stationary phases, the application of mixed-mode chromatographic separation are constrained. Innovative approaches are required to overcome these limitations and develop a pH stable and efficient mixed-mode stationary phase that offers enhanced selectivity, good peak shape and versatility [for charged analytes] while maintaining the integrity of the surface modification.

The listing or discussion of an apparently prior-published document in this specification should not necessarily be taken as an acknowledgement that the document is part of the state of the art or is common general knowledge.

### Description of the Invention

The present invention provides a method for making chromatographic packing material, wherein the packing material comprises a functionalised silicone-based polymer layer bonded to substrate particles and the method for forming the packing material comprises:
(a)
   (i) forming silicone-based polymer encapsulated substrate particles comprising pendant functional groups by reacting functional groups on the substrate particles with a silicone-based polymer product comprising pendant functional groups;
   (ii) functionalising the silicone-based polymer encapsulated substrate particles by reacting the pendant functional groups on the silicone-based polymer encapsulated substrate particles with at least one hydrophobic compound to form first functionalised silicone-based polymer encapsulated substrate particles; and
   (iii) functionalising the first functionalised silicone-based polymer encapsulated substrate particles by reacting the first functionalised silicone-based polymer encapsulated substrate particles with at least one amine containing compound to form second functionalised silicone-based polymer encapsulated substrate particles; or
(b)
   (i) forming a first functionalised silicone-based polymer by reacting pendant functional groups on a silicone-based polymer product with at least one hydrophobic compound;
   (ii) functionalising the first functionalised silicone-based polymer by reacting the first functionalised silicone-based polymer with at least one amine containing compound to form second functionalised silicone-based polymer; and
   (iii) reacting the second functionalised silicone-based polymer (product of step b (ii)) with functional groups on the substrate particles.

The substrate particles as defined herein, may be a particulate or monolithic substrate, preferably particulate.

The substrate material may be a metal oxide (which term herein includes a metalloid oxide, such as silica for example, and includes an inorganic-organic hybrid material (especially a metal oxide-organic hybrid material), as described in WO 00/45951 for example). The substrate may, in particular, be silica (SiOz), which term herein includes a silica/organo hybrid, alumina (Al₂ O₃), titania (TiO₂), or zirconia (ZrOz) substrate.

A silica (which term herein includes a silica/organo hybrid) substrate is most preferred.

The term "silicone-based" as used herein, is intended to cover any polymers and/or compounds that comprise silicone.

The term "functionalised silicone-based polymer layer" as used herein, is intended to cover a silicone-based polymer product to which additional compounds have been added which alter the functionality of the polymer. In particular, compounds such as hydrophobic compounds and amine containing compounds and compounds that provide further polymerisation.

The term "pendant" as used herein, is as defined in IUPAC nomenclature of chemistry, namely, a pendant group (sometimes spelled pendent) is a group of atoms attached to a backbone chain of a long molecule, usually a polymer. Pendant groups are different from pendant chains, as they are neither oligomeric nor polymeric.

The additional compounds may be single compounds or may themselves be monomers which either before or after addition to the silicone-based polymer layer form an additional polymeric layer bound to the silicone-based polymer. This is discussed in more detail below.

The functionalised silicone-based polymer layer is bound to the substrate particles. Typically, the functionalised silicone-based polymer layer is bound to the surface of the substrate particles, preferable through covalent bonds, i.e. the functionalised silicone-based polymer layer is covalently bonded to the substrate particles.

The functionalised silicone-based polymer bound to substrate particles may be formed by either step (a) or step (b) in the method of the invention.

### Step (a)

In step (a) (i), silicone-based polymer encapsulated substrate particles comprising pendant functional groups are formed by reacting functional groups on the substrate particles (as previously defined above) with a silicone-based polymer product comprising pendant functional groups.

Typically, the reaction between the functional groups on the substrate particles with a silicone-based polymer product comprising pendant functional groups forms a covalent bond.

The functional groups on the substrate particles may preferably be selected from hydroxyl, epoxy and thiol. In a most preferred aspect, the functional groups are hydroxyl groups.

The functional groups on the substrate particles (that are reacted with the silicone-based polymer product comprising pendant functional groups) are typically located on the surface of the substrate particles. For example, the silicone-based polymer product comprising pendant functional groups may be reacted with functional groups, i.e. hydroxyl groups, on the surface of the substrate particles.

The silicone-based polymer product preferably comprises and utilises a silyl group or groups for attaching the polymer to the substrate (especially silica substrate). The silyl group accordingly is preferably an activated silyl group, i.e. having groups (leaving groups) that can react with a substrate (especially silica) surface and enable attachment of the polymer to the substrate surface. The silyl groups in this way may covalently bond the polymer to the silica substrate via siloxane bonds (Si-O-Si). A first Si atom in the siloxane bond is derived from the silyl group. A second Si atom in the siloxane bond is derived from the silica, i.e. the silica surface.

The silyl group or groups of the polymer molecules may preferably have a formula: 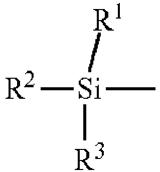 wherein at least one of R¹, R², R³ is a leaving group. Preferably, R¹, R², R³ are independently selected from an oxygen atom (e.g. that connects to a substrate (silicon) atom in the substrate or connects to another silicon atom of the polymer), a hydroxyl group, a halogen atom, an alkoxy group (i.e. methoxy, ethoxy, etc), a dialkylamino group, an acyl group, an alkyl group (optionally a heteroalkyl group or a heterocycloalkyl group), an aryl group (optionally a heteroaryl group), or a pendant functional group (reactive group) (i.e. the first reactive group described herein, for example an olefinic group, such as vinyl, allyl etc.).

The groups R¹, R², and R³ may be the same or all different. Preferably, at least one, optionally two of R¹, R², R³ groups is a leaving group. More preferably, at least one of R¹, R², R³ groups is an alkoxy group (preferably methoxy, ethoxy or propoxy, especially methoxy), a dialkylamino group, or a halogen atom.

The silicone-based polymer product also comprises multiple pendant functional groups, also referred to hereinafter as first reactive groups.

The pendant functional groups (first reactive groups) may preferably be reactive olefinic groups or reactive thiol groups, especially olefinic groups. The reactive olefinic groups of the polymer may preferably be vinyl or allyl groups. The multiple reactive groups may preferably be all of the same type, e.g. all vinyl.

The pendant functional groups may be groups that are capable of participating in a radical polymerisation reaction. For example, the pendant functional groups capable of participating in a radical polymerisation reaction may be vinyl, allyl, methacrylate, acrylate, acrylamide, methacrylamide and styrene groups, preferably vinyl groups.

As used herein, the term "capable of participating in a radical polymerisation reaction" is intended to mean that the group(s) when in the presence of an initiator will form a radical that will react with other polymerizable groups to form either a polymer chain (i.e. a homopolymer or a copolymer chain), or result in crosslinking.

The polymer may preferably have a "fixed" distance between adjacent first reactive groups (e.g. between adjacent vinyl groups), i.e. the distance between adjacent first reactive groups is substantially uniform for all the reactive groups in the polymer molecule.

The silicone-based polymer product may preferably comprise a vinyl siloxane polymer, (i.e. a siloxane polymer having reactive vinyl groups). The size (i.e. number average molecular weight, MWn) of the (optionally vinyl) siloxane polymer is preferably 500-10,000 daltons (Da). The vinyl siloxane polymer is preferably a vinylalkoxysiloxane polymer
In the case of vinylsiloxane polymers, typically the silyl group has a leaving group (terminal silyl groups may have two leaving groups), a reactive group and two bonds (terminal silyl groups have one such bond) to respective oxygen atoms that are connected to adjacent silicon atoms of the polymer). The leaving groups are capable of allowing the polymer to form the siloxane bonds to the substrate silica surface.

The vinyl siloxane polymer may have a formula I: 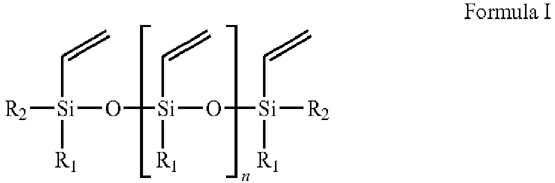 wherein n is an integer from 3 to 100 R₁, and R₂ are independently selected from alkoxy, especially methoxy and ethoxy, hydroxyl and halo (especially CI). R₁ and R₂ are preferably independently selected from alkoxy, especially methoxy and ethoxy, and hydroxyl. R₁ and R₂ are more preferably independently selected from alkoxy, especially methoxy and ethoxy. R₁ and R₂ are especially preferably the same, preferably both either methoxy or ethoxy.

Alternatively, the vinyl siloxane polymer may have a formula II:

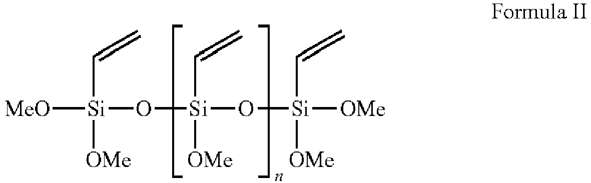

Alternatively, the silicone-based polymer product may have ethoxy groups, or hydroxyl groups, in place of the methoxy groups in formula II.

The silicone-based polymer product (such as a vinyl siloxane polymer) may be a co-polymer, e.g. containing a mixture of vinyl siloxane units and alkyl siloxane units (especially C₁-C₄ alkyl). The co-polymer may have a nominal formula III for example: 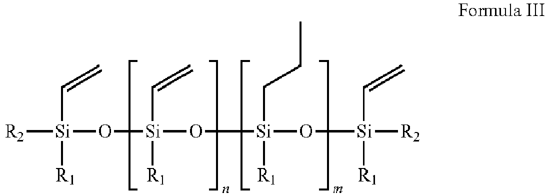 wherein R₁, and R₂ are defined as above for formulae I and II and wherein n is an integer from 3 to 100 and m is an integer from 1 to 70 (preferably 1 to 20), or formula IV for example.

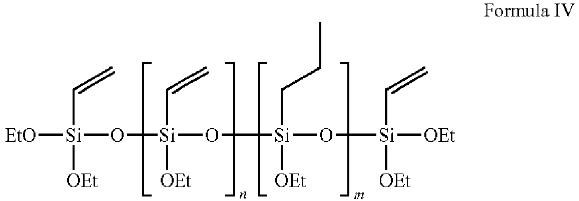

The vinyl siloxane units and the alkyl siloxane units in the above formulae may be present in the polymer as blocks, or randomly distributed, or in alternating positions. Alternatively, the polymer may have methoxy groups, or hydroxyl groups, in place of the ethoxy groups in formula IV.

The silicone-based polymer product may comprise at least one silyl group containing polymer (i.e. at least one polymer containing multiple silyl groups), preferably having 500-10,000 dalton MWn. In particular, such polymer may be modified polybutadiene, especially silyl modified polybutadiene, preferably having 500-10,000 dalton MWn.

The silicone-based polymer product may preferably comprise trialkoxysilyl groups as the silyl groups (e.g. trimethoxysilyl or triethoxysilyl). The at least one silyl modified polybutadiene may preferably comprise a trialkoxysilyl modified polybutadiene (the size of the trialkoxysilyl modified polybutadiene polymer (i.e. molecular weight, MWn) is preferably 500-10,000 dalton). For example, the polybutadiene may be a trimethoxysilyl modified polybutadiene or a triethoxysilyl modified polybutadiene.

The silyl modified polybutadiene may have a nominal repeat unit of formula V: 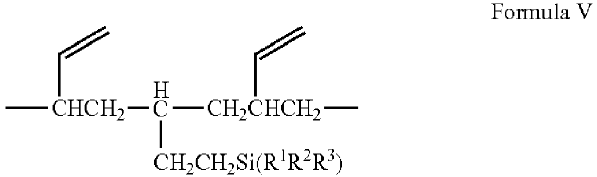 wherein R¹, R² and R³ are independently selected from alkoxy, especially methoxy and ethoxy, hydroxyl, halo (especially Cl) and alkyl (especially C₁-C₃ alkyl, more especially methyl), provided that at least one of R¹, R² and R³ is a leaving group (especially methoxy or ethoxy). R¹, R² and R³ are preferably independently selected from alkoxy, especially methoxy and ethoxy, and hydroxyl. R¹, R² and R³ are more preferably independently selected from alkoxy, especially methoxy and ethoxy R¹, R² and R³ are especially preferably the same, preferably both either methoxy or ethoxy.

The silyl modified polybutadiene may be an alkoxysilyl modified polybutadiene having a nominal repeat unit of formula VI: 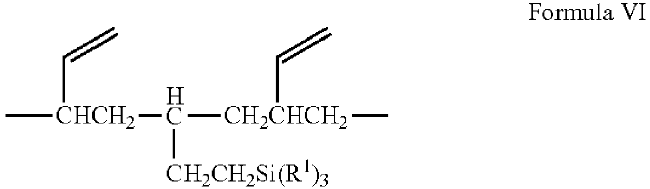 wherein each R¹ is independently either methoxy or ethoxy. Preferably all R¹ are the same group.

For example:

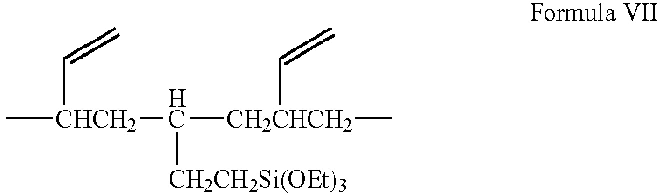

Alternatively, the polymer may have methoxy groups, or hydroxyl groups, in place of the ethoxy groups in formula VII.

In another preferred embodiment, the silyl modified polybutadiene may be an alkylalkoxysilyl modified polybutadiene. For example, the alkylalkoxysilyl modified polybutadiene may have a nominal repeat unit of formula VIII:

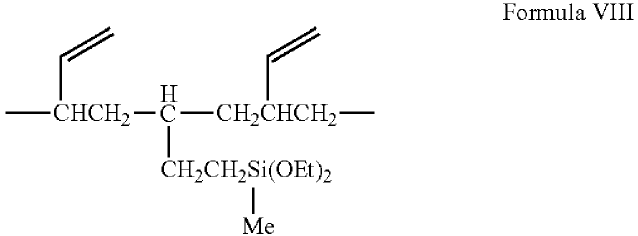

Alternatively, the polymer may have methoxy groups, or hydroxyl groups, in place of the ethoxy groups in formula VIII.

After reaction with functional groups on the substrate particles, such as the surface of the substrate particles (i.e. step (a) (i), the silicone-based polymer product now encapsulates the substrate particles forming the silicone-based polymer encapsulated substrate particles comprising pendant functional groups.

An example of the reaction between the functional groups on the substrate particles and a polymer of formula I (having methoxy and ethoxy groups) and pendent vinyl reactive groups (vinyl pendant functional groups) are present is shown below (Scheme 1):

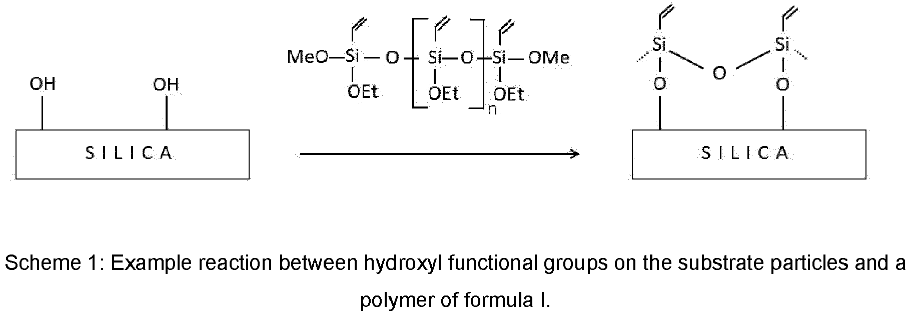

In the formula I or II, any number of the vinyl groups may be replaced by alkyl groups, e.g. C₁-C₄ alkyl, especially C₁-C₃ alkyl.

A covalent attachment of the polymer of formula V, VI or VII to silica may yield a silicone-based polymer encapsulated substrate particles comprising pendant functional groups where the silicone-based polymer product is bound to the surface as show:

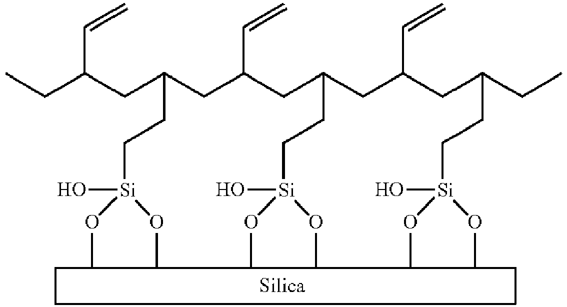

A covalent attachment of the polymer of formula VIII may yield a silicone-based polymer encapsulated substrate particles comprising pendant functional groups where the silicone-based polymer product is bound to the surface as show:

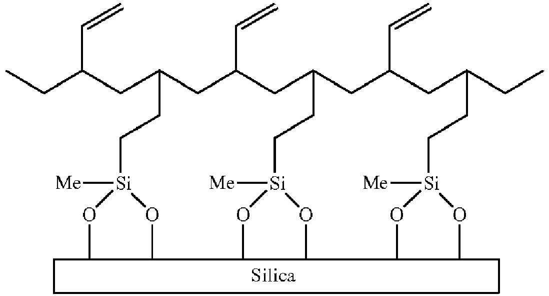

The use of the polymers described herein provide numerous benefits, for example: allowing multiple attachment to the substrate for stability whilst being controllable compared to very high MW polymers that may tend to clog pores in the substrate; allowing for subsequent surface modification with copolymerization of allyl or vinyl functional compounds, wherein a "fixed" distance between adjacent reactive (e.g. vinyl) groups favours the formation of a more uniform protective layer on the substrate surface; and allowing flexibility in the stationary phase or column chemistry due to a choice of functional groups for attaching to the polymer.

After formation of the silicone-based polymer encapsulated substrate particles comprising pendant functional groups as detailed above in Step (a) (i), the encapsulated particles are then further reacted in Step (a) (ii) in order to functionalise the silicone-based polymer layer and form first functionalised silicone-based polymer encapsulated substrate particles.

In Step (a) (ii), this is achieved by reacting the pendant functional groups on the silicone-based polymer encapsulated particles with at least one hydrophobic compound.

After Step (a) (i), pendant functional groups are located on the surface of the silicone-based polymer encapsulated substrate particles, that is in Step (a) (ii), functionalising the silicone-based polymer encapsulated substrate particles may comprise reacting pendant functional groups located on the surface of the silicone-based polymer encapsulated substrate particles with at least one hydrophobic compound to form first functionalised silicone-based polymer encapsulated substrate particles.

This helps to provide fuller coverage of the substrate particle surface with reactive groups.

The at least one hydrophobic compound typically comprises at least one group that is reactive with the pendant functional groups on the silicone-based polymer encapsulated substrate particles, for example, the at least one hydrophobic compound may comprise one, two or three groups that are reactive with the pendant functional groups located on the silicone-based polymer encapsulated substrate particles. The group may be selected from the group consisting of a vinyl group, an allyl group and/or a thiol group.

In a preferred aspect, the at least one hydrophobic compound may be a branched or unbranched C₄ to C₃₀ alkyl. In an aspect of the invention, the at least one branched or unbranched C₄ to C₃₀ alkyl may be monounsaturated, i.e. comprise a double bond. For example, the at least one branched or unbranched C₄ to C₃₀ alkyl may preferably comprise a terminal double bond (vinyl group). In a preferred aspect, the C₄ to C₃₀ alkyl is unbranched and monounsaturated, in particular where the monounsaturation is terminal (i.e. vinyl).

Examples of the at least one hydrophobic compound include, but are not limited to regio-isomers of butene, pentene, hexene, octene, nonene, decene, undecene, dodecene, tridecene, tetradecene, pentadecene, hexadecene, heptadecene, octadecene, nonadecene, Eicosene, Heneicosene, docosene, tricosene, tetracosene, pentacosene, hexacosene, heptacosene, octocosene, nonacosene and tricontene, such as 1-butene, 1-pentene, 1-hexene, 1-heptene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-Eicosene, 1-Heneicosene, 1-docosene, 1-tricosene, 1-tetracosene, 1-pentacosene, 1-hexacosene, 1-heptacosene, 1-octocosene, 1-nonacosene and 1-tricontene.

The at least one hydrophobic compound typically reacts with the pendant functional groups in a radical polymerisation reaction such that the first functionalised silicone-based polymer encapsulated substrate particles has an additional polymeric layer bonded to the silicone-based polymer layer, i.e. it comprises the silicone-based polymer layer surrounding the substrate particles and an additional first functionalised polymer layer surrounding/bonded to the silicone-based polymer layer.

This may also be referred to hereinafter as the first additional polymer layer.

That is step (a) (ii) may comprise reacting the pendant functional groups on the silicone-based polymer encapsulated particles with at least one hydrophobic compound in a radical polymerisation reaction to form first functionalised silicone-based polymer encapsulated substrate particles (which comprise a first additional polymer layer).

Thus, step (a) (ii) may comprise functionalising the silicone-based polymer encapsulated substrate particles by forming first functionalised silicone-based polymer encapsulated substrate particles with a first additional polymer layer by reacting the pendant functional groups on the silicone-based polymer encapsulated substrate particles with at least one hydrophobic compound in a radical polymerisation reaction.

Alternatively, step (a) (ii) may comprise further encapsulation of the silicone-based polymer encapsulated substrate particles by reacting the pendant functional groups on the silicone-based polymer encapsulated substrate particles with at least one hydrophobic compound in a radical polymerisation reaction forming first functionalised silicone-based polymer encapsulated substrate particles.

An example reaction between silicone-based polymer encapsulated substrate particles and at least one hydrophobic compound is shown below (Scheme 2):

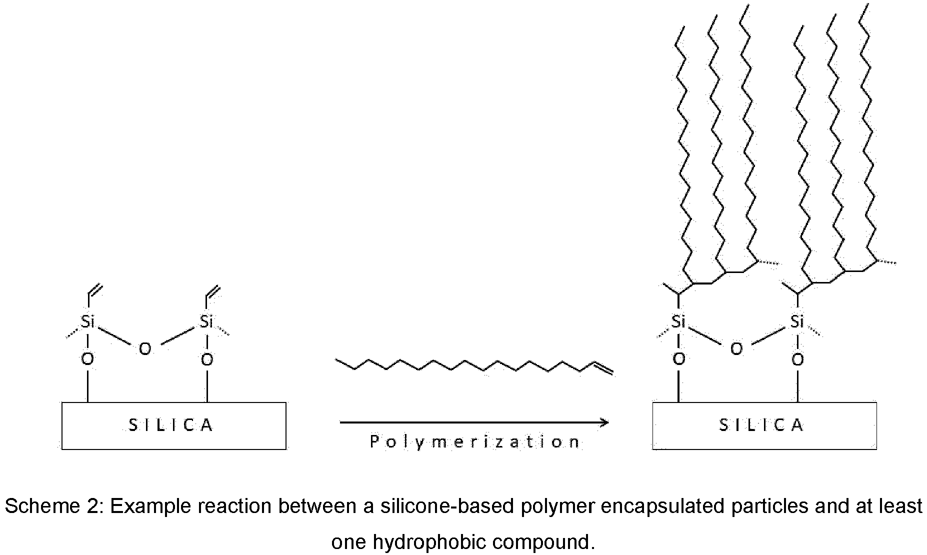

In step (a) (iii), the first functionalised silicone-based polymer encapsulated substates particles are further functionalised by reacting the first functionalised silicone-based polymer encapsulated substates particles with at least one amine containing compound to form second functionalised silicone-based polymer encapsulated substrate particles.

The amine of the at least one amine containing compound may be a primary, secondary or tertiary amine.

The at least one amine containing compound may also or alternatively be a polymerizable amine containing compound, i.e. an amine containing monomer. For example, the at least one amine containing compound may be a polymerizable primary, secondary or tertiary amine containing monomer.

In the at least one amine containing compound/monomer, the amine may preferably be a tertiary amine.

The at least one amine containing compound/monomer may preferably only comprise amine functional groups. That is the at least one amine containing compound/monomer may only comprise carbon, hydrogen and nitrogen atoms.

The at least one amine containing compound/monomer as defined above, may comprise more than one amine group, for example, the at least one amine containing compound may comprise two, three, four or five amine groups, which individually may be primary, secondary or tertiary amine groups.

Examples of the at least one polymerizable amine containing compound include, but are not limited to, N,N-Diisopropylethylamine, tetramethylethylenediamine, N,N,N',N",N"-pentamethyldiethylenetriamine.

As with step (a) (ii), the reaction between the first functionalised silicone-based polymer encapsulated substates particles and the at least one amine containing compound may be a radical polymerisation reaction.

That is, in step (a) (iii) a further polymer layer may be formed either around and/or bonded to the first functionalised silicone-based polymer encapsulated substates particles. This may be referred to as the second additional polymer layer.

Alternatively, or additionally, the radical polymerisation reaction may result in crosslinking of the first additional polymer layer formed on the first functionalised silicone-based polymer encapsulated substates particles.

Thus, the second functionalised silicone-based polymer encapsulated substates particles may comprise a first and second additional polymer layer, wherein the first and/or second polymer layer may be crosslinked or may comprise a first additional polymer layer with additional crosslinking.

That is step (a) (iii) may comprise reacting the first functionalised silicone-based polymer encapsulated substates particles with at least one at least one amine containing compound in a radical polymerisation reaction to form second functionalised silicone-based polymer encapsulated substrate particles (which comprise additional crosslinking in the first additional polymer layer or comprise a second additional polymer layer, wherein the first and/or second additional polymer layers may or may not be crosslinked).

Thus, step (a) (iii) may include functionalising the first functionalised silicone-based polymer encapsulated substates particles by forming second functionalised silicone-based polymer encapsulated substrate particles by crosslinking the first additional polymer layer or by the addition of a second additional polymer layer, wherein the first and/or second additional polymer layers may or may not be crosslinked, by reacting first functionalised silicone-based polymer encapsulated substates particles with at least one at least one amine containing compound in a radical polymerisation reaction.

Alternatively, step (a) (iii) may include further encapsulation of the first functionalised silicone-based polymer encapsulated substates particles by reacting the first functionalised silicone-based polymer encapsulated substates particles with at least one at least one amine containing compound in a radical polymerisation reaction forming second functionalised silicone-based polymer encapsulated substrate particles (which comprise additional crosslinking in the first additional polymer layer or comprise a second additional polymer layer, wherein the first and/or second additional polymer layers may or may not be crosslinked).

An example reaction between first functionalised silicone-based polymer encapsulated substrate particles and at least one amine containing compound is shown below (Scheme 3):

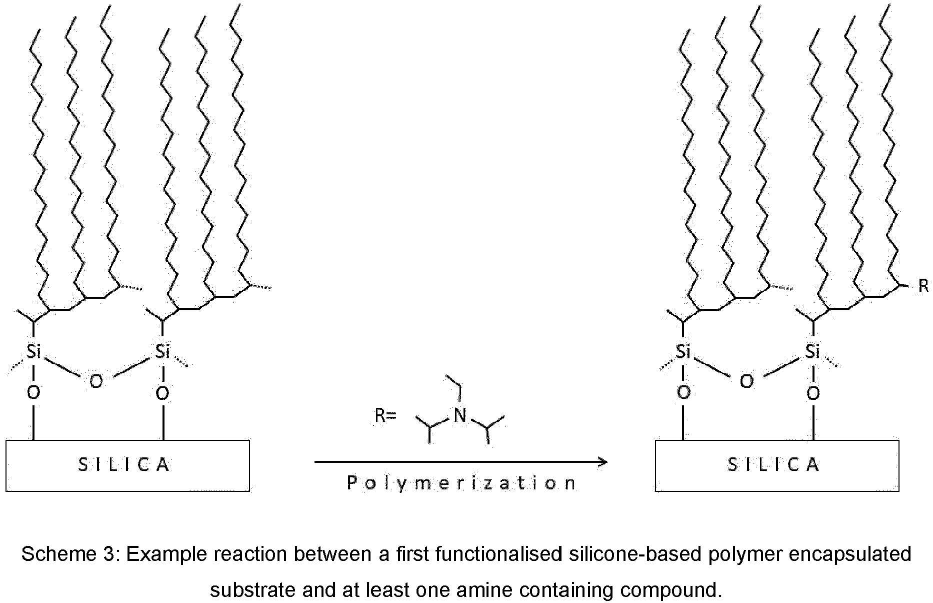

Step (a) (i) of the method of the invention may typically be performed at a temperature of from about 20° C to about 250° C, preferably from about 100° C to about 225° C, such as about 200° C.

Typically, step (a) (i) of the method may be conducted from about 2 to about 72 hours, preferably from about 24 to about 64 hours, such as about 48 hours.

Step (a) (i) may be conducted in the presence of a solvent. The solvent may preferably be a water immiscible organic solvent such as toluene, ethylbenzene, dimethylbenzene, n-hexane, n-heptane, n-pentane, n-octane, or cyclohexane. The solvent may preferably be toluene.

Alternatively, step (a) (i) of the method may be conducted under reduced pressure, such as below 500 mbar.

Where step (a) (i) is conducted at a reduced pressure, it may be preferred that the step is conducted in the absence of solvent.

Steps (a) (ii) and/or (iii) of the method of the invention may also preferably be conducted under elevated temperature and/or reduced pressure in the absence of solvent.

It may be preferred that steps (a) (ii) and/or (iii) are conducted in the presence of an initiator to promote free radical polymerisation.

The elevated temperature for steps (a) (ii) and/or (iii) may preferably be at least about 100 ° C, or at least about 110 ° C, or at least about 120 ° C, or at least about 140 ° C. For example, the elevated temperature for step (a) (ii) may be up to about 300 ° C, or up to about 200 ° C, or up to about 190 °C, or up to about 180 ° C, or up to about 160 ° C.

More preferably, the elevated temperature for steps (a) (ii) and/or (iii) may be from about 100 ° C to about 300 ° C, or from about 100 ° C to about 200 ° C, or from about 110 ° C to about 190 ° C, or from about 120 ° C to about 180 ° C, or from about 130 ° C to about 170 ° C.

The reduced pressure for step (a) (i), (ii) or (iii) when used may be preferably less than 500 mbar, more preferably less than 400 mbar, still more preferably less than 300 mbar, yet still more preferably less than 200 mbar, and most preferably less than 100 mbar.

For example, the pressure may be preferably at least 0.01 mbar, more preferably at least 0.1 mbar, or at least 1 mbar.

The reduced pressure and the elevated temperature for step (a) (i), (ii) or (iii) when used may be preferably applied concurrently at least for a period of time (first reactions period).

This period of time may be at least 1 hour, or at least 2 hours, or at least 4 hours, or at least 8 hours, or at least 12 hours. The period of time may be up to 20 hours, or up to 30 hours.

The reduced pressure is preferably applied for substantially the same period as the elevated temperature.

Steps (a) (i), (ii) and (iii) may be preferably performed in an inert atmosphere, i.e. in an inert gas (e.g. nitrogen, or argon). The reactants are preferably purged with inert gas before applying the elevated temperature and, in the case of reaction at reduced pressure, before the procedure.

In step (a) (i), the ratio of substrate particles to silicone-based polymer may be from about 10:1 to about 1:10, such as from about 6:1 to about 1:6.

In steps (a) (ii) and (iii), the ratio of silicone-based polymer encapsulated substrate particles and the hydrophobic compound (step (a) (ii)) or the ratio of first functionalised silicone-based polymer encapsulated substates particles and the at least one amine containing compound compound (step (a) (iii) may be from about 1:1 to about 200:1, such as from about 2:1 to about 8:1, such as about 4:1.

As detailed above, the functionalised silicone-based polymer bound to substrate particles may be formed the either step (a) or step (b) in the method of the invention.

### Step (b)

In step (b) (i), a first functionalised silicone-based polymer is formed by reacting pendant functional groups on a silicone-based polymer product with at least one hydrophobic ligand.

In step (b) (ii), the first functionalised silicone-based polymer is further functionalised by reacting the first functionalised silicone-based polymer with at least one amine containing compound to form second functionalised silicone-based polymer.

Performing Step (b) (i) and (ii) forms a functionalised silicone-based polymer before the substrate particles are encapsulated, i.e. before the substrate particles and polymer are joined.

As with Step (a) (ii) above, the reaction between the pendant functional groups on the silicone-based polymer product in step (b) (i) and the at least one hydrophobic compound may form an additional polymer layer bonded to the silicone-based polymer product. This may also be referred to hereinafter as the first additional polymer layer.

That is step (b) (i) may comprise reacting the pendant functional groups on the silicone-based polymer product with at least one hydrophobic compound in a radical polymerisation reaction.

Thus, step (b) (i) may include functionalising the silicone-based polymer product by forming a first additional (or further) polymer layer by reacting the pendant functional groups on the silicone-based polymer encapsulated substrate particles with at least one hydrophobic compound in a radical polymerisation reaction.

In step (b) (ii), as with step (b) (i), the reaction between the first functionalised silicone-based polymer and the at least one amine containing compound may be a radical polymerisation reaction.

That is, in step (b) (ii) a further polymer layer may be formed on the first functionalised silicone-based polymer. This may be referred to as the second additional polymer layer.

Alternatively, or additionally, the radical polymerisation reaction may result in crosslinking of the polymer formed on the first functionalised silicone-based polymer.

Thus, the second functionalised silicone-based polymer may comprise a first and second additional polymer layer, wherein the first and/or second polymer layer may be crosslinked or may comprise a first additional polymer layer with additional/further crosslinking.

That is step (b) (ii) may comprise reacting the first functionalised silicone-based polymer with at least one amine containing compound in a radical polymerisation reaction to form second functionalised silicone-based polymer (which comprise additional crosslinking in the first additional polymer layer or comprise a second additional polymer layer, wherein the first and/or second additional polymer layers may or may not be crosslinked).

Thus, step (b) (ii) may include functionalising the first functionalised silicone-based polymer by forming second functionalised silicone-based polymer by crosslinking the first additional polymer layer or by the addition of a second additional polymer layer, wherein the first and/or second additional polymer layers may or may not be crosslinked, by reacting the first functionalised silicone-based polymer with at least one amine containing compound in a radical polymerisation reaction.

Alternatively, step (b) (ii) may include further encapsulation of the first functionalised silicone-based polymer by reacting the first functionalised silicone-based polymer with at least one amine containing compound in a radical polymerisation reaction forming second functionalised silicone-based polymer (which comprise additional crosslinking in the first additional polymer layer or comprise a second additional polymer layer, wherein the first and/or second additional polymer layers may or may not be crosslinked).

Encapsulating the substrate particles occurs in Step (b) (iii) where the functionalised silicone-based polymer (i.e. the product of Step (b) (i)) is reacted with functional groups on the substrate particles.

The functional groups on the substrate particles, the substrate particles, the silicone-based polymer product, the pendant functional groups on the silicone-based polymer product, the at least one hydrophobic ligand and the at least one amine containing ligand are as defined for Step (a) above.

It should be noted that in any of the polymerisation reaction, any suitable polymerisation initiator may be used. The initiator will depend on the compounds present and the skilled person would be able to ascertain which would be most suitable.

Additionally, the temperatures, pressures and ratios defined above with respect to step (a), may be used for the corresponding step in step (b). That is, the reaction between the silicone-based polymer product and the hydrophobic compound in step (b) (i) and the reaction between the first functionalised silicone-based polymer product in step b (ii) may utilise the same reaction parameters as used in step (a) (ii).

In a preferred aspect, in the method for making chromatographic packing material, wherein the packing material comprises a functionalised silicone-based polymer layer bonded to substrate particles, the method for forming the packing material may comprise:
(a)
   (i) forming silicone-based polymer encapsulated substrate particles comprising pendant functional groups by reacting hydroxyl, epoxy and/or thiol functional groups on the substrate particles with a silicone-based-based polymer product comprising pendant functional groups, wherein the silicone-based polymer product is a vinylsiloxane with the formula 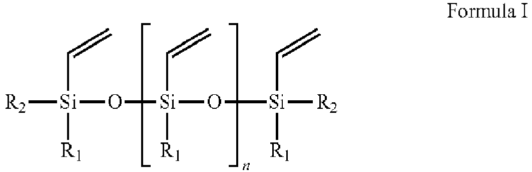 wherein n is an integer from 3 to 100, R₁ and R₂ are independently selected from the group consisting of: alkoxy, hydroxyl and halo; and
   (ii) functionalising the silicone-based polymer encapsulated substrate particles by reacting the pendant vinyl functional groups on the silicone-based polymer encapsulated substrate particles with at least one monounsaturated, unbranched C₄ to C₃₀ alkyl compound to form first functionalised silicone-based polymer encapsulated particles ;
   (ii) functionalising the first functionalised silicone-based polymer encapsulated substrate particles by reacting the first functionalised silicone-based polymer encapsulated substrate particles with at least one amine containing compound to form second functionalised silicone-based polymer encapsulated substrate particles; or
(b)
   (i) forming a first functionalised silicone-based polymer by reacting pendant vinyl functional groups on a silicone-based polymer product, wherein the silicone-based polymer product is a vinylsiloxane with the formula 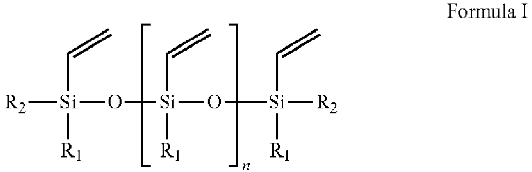 wherein n is an integer from 3 to 100, R₁ and R₂ are independently selected from the group consisting of: alkoxy, hydroxyl and halo with at least one monounsaturated, unbranched C₄ to C₃₀ alkyl compound;
   (ii) functionalising the first functionalised silicone-based polymer by reacting the first functionalised silicone-based polymer with at least one amine containing compound to form second functionalised silicone-based polymer; and
   (iii) reacting the product of step b (ii) with hydroxyl, epoxy and/or thiol functional groups on the substrate particles.

The resulting chromatographic packing material is provided in a form suitable for use as chromatographic packing.

Thus, the present invention also provides chromatographic packing material formed by the method of the invention as defined above.

The present invention also provides chromatographic packing material comprising:
(i) Substrate particles;
(ii) A functionalised silicone-based polymer layer, bonded to the substrate particles, prepared by:
   (a)
      (i) forming silicone-based polymer encapsulated substrate particles comprising pendant functional groups by reacting functional groups on the substrate particles with a silicone-based polymer product comprising pendant functional groups;
      (ii) functionalising the silicone-based polymer encapsulated substrate particles by reacting the pendant functional groups on the silicone-based polymer encapsulated substrate particles with at least one hydrophobic compound to form first functionalised silicone-based polymer encapsulated substrate particles; and
      (iii) functionalising the first functionalised silicone-based polymer encapsulated substrate particles by reacting the first functionalised silicone-based polymer encapsulated substrate particles with at least one amine containing compound to form second functionalised silicone-based polymer encapsulated substrate particles; or
   (b)
      (i) forming a first functionalised silicone-based polymer by reacting pendant functional groups on a silicone-based polymer product with at least one hydrophobic compound;
      (ii) functionalising the first functionalised silicone-based polymer by reacting the first functionalised silicone-based polymer with at least one amine containing compound to form second functionalised silicone-based polymer; and
      (iii) reacting the product of step b (ii) with functional groups on the substrate particles.

This may hereinafter be referred to as the chromatographic packing material of the invention or the packing material of the invention.

In the packing material of the invention, the bond between the functionalised silicone-based polymer and the substrate particles may be a covalent bond.

As detailed above with regards the method of the invention, this is because the bond may typically be formed by reaction between a hydroxyl, epoxy and/or thiol functional group on the substrate particles (for example, the surface of the substrate particles) and a leaving group, such as an alkoxy group present on the silicone-based polymer product.

The functional groups on the substrate particles, the substrate particles, the silicone-based polymer product, the pendant functional groups on the silicone-based polymer product, the at least one hydrophobic ligand and the at least one amine containing ligand are as defined for the method of the invention above.

That is in the chromatographic packing material the functional groups on the substrate particles may be selected from hydroxyl, epoxy and thiol as previously described.

The substrate particles may be selected from the group consisting of metal oxides and organic polymers as previously described.

The pendant functional groups on the silicone-based polymer encapsulated substrate particles or the silicone-based polymer may typically comprise groups capable of participating in a radical polymerisation reaction as previously described.

The pendant functional groups on the silicone-based polymer encapsulated substrate particles or the silicone-based polymer may be reactive olefinic groups or reactive thiol groups, preferably olefinic groups as previously described.

The reactive olefinic groups or reactive thiol groups may be selected from vinyl, allyl, methacrylate, acrylate, acrylamide, methacrylamide and styrene as previously described.

The silicone-based polymer product typically comprises at least one leaving group as previously described. For example, the at least one leaving group may be an alkoxy group.

The silicone-based polymer product may comprise or consist of vinylsiloxane.

The vinylsiloxane polymer may typically have the formula described above in the method of the invention.

As described in the method of the invention the reaction between the pendant functional groups on the silicone-based polymer encapsulated substrate particles in step (a) (ii) and the at least one hydrophobic compound may form an additional polymer layer bonded to the silicone-based polymer encapsulated substrate particles. This reaction may be a free-radical polymerisation reaction.

Further, the reaction between the first functionalised silicone-based polymer encapsulated substrate particles and the at least one amine containing compound in step (a) (iii) may be a free-radical polymerisation that may form an additional polymer layer bonded to the first functionalised silicone-based polymer encapsulated substrate particles. Alternatively, the free-radical polymerisation may form crosslinking in the additional polymer layer bonded to the silicone-based polymer encapsulated substrate particles. In some instances, both an additional polymer layer and crosslinking may occur.

The reaction between the pendant functional groups on the silicone-based polymer product in step (b) (i) and the at least one hydrophobic compound forms an additional polymer layer bonded to the silicone-based polymer product. This reaction may be a free-radical polymerisation reaction.

The reaction between the first functionalised silicone-based polymer and the at least one amine containing compound in step (b) (ii) may also be a free-radical polymerisation and may form an additional polymer layer bonded to the first functionalised silicone-based polymer. Alternatively, the free-radical polymerisation may form crosslinking in the first functionalised silicone-based polymer. In some instances, both an additional polymer layer and crosslinking may occur.

In a preferred aspect, the chromatographic packing material of the invention may be provided by the method of the invention as defined herein.

The ratio of silicone-based polymer encapsulated substrate particles and the hydrophobic compound and/or the ratio of first functionalised silicone-based polymer encapsulated substates particles and the at least one amine containing compound may be from about 0:1 to about 50:1, such as from about 2:1 to about 8:1, such as about 4:1.

In a preferred aspect, the present invention provides chromatographic packing material comprising:
(i) Metal oxide or organic polymer substrate particles, preferably silica substrate particles, comprising hydroxyl, epoxy and/or thiol functional groups;
(ii) A functionalised silicone-based polymer layer, covalently bonded to the substrate particles, prepared by:
   (a)
      (i) forming silicone-based polymer encapsulated substrate particles comprising pendant functional groups by reacting hydroxyl, epoxy and/or thiol functional groups on the substrate particles with a silicone-based-based polymer product comprising pendant functional groups, wherein the silicone-based polymer product is a vinylsiloxane with the formula 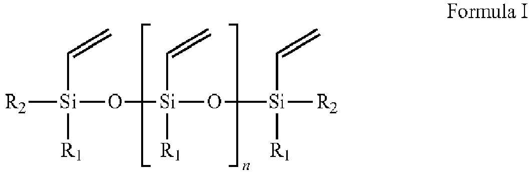 wherein n is an integer from 3 to 100, R₁ and R₂ are independently selected from the group consisting of: alkoxy, hydroxyl and halo; and
      (ii) functionalising the silicone-based polymer encapsulated substrate particles by reacting the pendant vinyl functional groups on the silicone-based polymer encapsulated substrate particles with at least one monounsaturated, unbranched C₄ to C₃₀ alkyl compound to form first functionalised silicone-based polymer encapsulated particles ;
      (ii) functionalising the first functionalised silicone-based polymer encapsulated substrate particles by reacting the first functionalised silicone-based polymer encapsulated substrate particles with at least one amine containing compound to form second functionalised silicone-based polymer encapsulated substrate particles; or
   (b)
      (i) forming a first functionalised silicone-based polymer by reacting pendant vinyl functional groups on a silicone-based polymer product, wherein the silicone-based polymer product is a vinylsiloxane with the formula 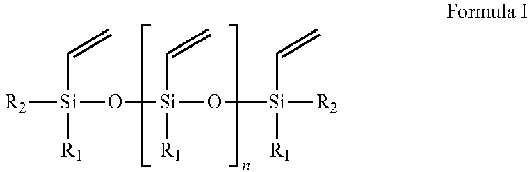 wherein n is an integer from 3 to 100, R₁ and R₂ are independently selected from the group consisting of: alkoxy, hydroxyl and halo with at least one monounsaturated, unbranched C₄ to C₃₀ alkyl compound;
      (ii) functionalising the first functionalised silicone-based polymer by reacting the first functionalised silicone-based polymer with at least one amine containing compound to form second functionalised silicone-based polymer; and
(iii) reacting the product of step b (ii) with hydroxyl, epoxy and/orthiol functional groups on the substrate particles. In a particular aspect, the packing material may be obtained using a method as defined herein.

The packing material of the invention may be used in chromatographic separation.

The specific combination of at least one hydrophobic compound and at least one amine containing compound in the additional polymer layer has surprisingly and unexpectedly been shown to provide particularly effective hydrophobic retention and ion exchange selectivity, with exceptional selectivity for glycosylated and deaminated peptides. As well as providing protection against low pH and acidic aqueous mobile phases.

Thus, the present invention also provides the use of packing material as defined herein in chromatographic separation, in particular, the separation of glycopeptides.

In an aspect of the invention, the use in chromatographic separation may be preferred when conducted using a mobile phase with a pH 1.0 or less or conducted using a mobile phase with a pH 13.0 or more.

For example, the use in the separation of glycopeptides using a mobile phase with a pH 1.0 or less or using a mobile phase with a pH 13.0 or more.

For the avoidance of doubt, in this specification when we use the term "comprising" or "comprises" we mean that the feature being described must contain the listed component(s) but may optionally contain additional components. When we use the term "consisting essentially of' or "consists essentially of' we mean that the feature being described must contain the listed component(s) and may also contain other components provided that any components do not affect the essential properties of the feature. When we use the term "consisting of' or "consists of' we mean that the feature being described must contain the listed component(s) only.

As used herein, the term "at least one" is intended to mean that the compound comprises one or more of the features referred to. In particular, one, two, three or four of the features referred to, from one to four or from one to three.

It would be clear to the person skilled in the art that the features and combinations defined with respect to the method of the invention, apply equally to the packing material.

The detailed description illustrates, by way of example, not by way of limitation, the principles of the invention. The description will clearly enable one skilled in the art to make and use the invention, and described several embodiments, adaptions, variations, alternatives and uses of the invention. As used herein, the terms "about" or "approximately" for any numerical values or ranges indicate a suitable dimensional tolerance that allows the part or collection of components to functions for its intended purpose as described.

### Brief Description of the Drawings

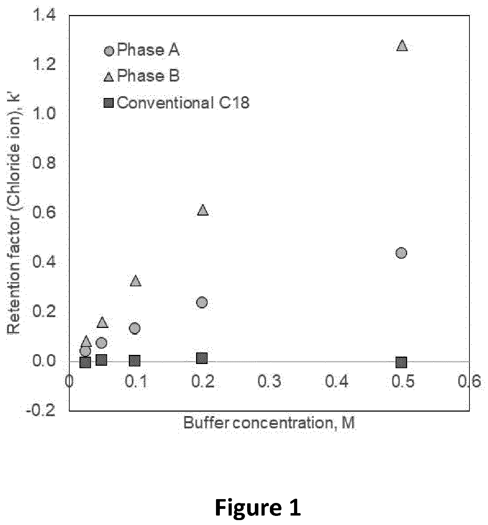

Figure 1: The ion exchange test results obtained with CAD detector with ammonium chloride as a sample (mobile phase A: 90% 100mM ammonium formate + 10% acetonitrile, pH 3; mobile phase B: 90% DI water + 10% acetonitrile). (Grey circle) Phase A (this invention), (green triangle) Phase B (this invention), (blue square) conventional C18 packing under the same testing conditions.

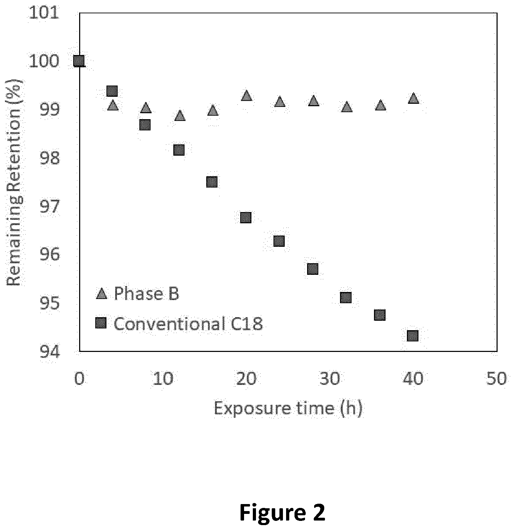

Figure 2: Dependence of the percentage change in the retention time of acenaphthene on the time exposed to 0.1% TFA at 50 °C for (green triangles) Phase B and (blue squares) conventional C18 packing under the same testing conditions.

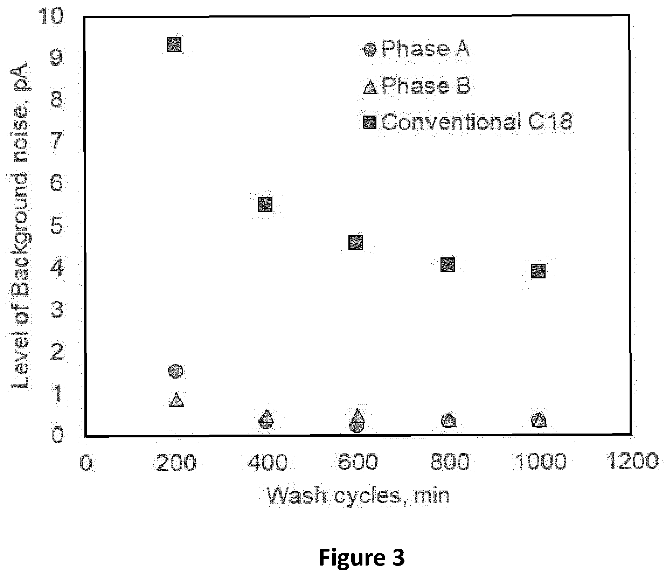

Figure 3: Dependence of detector response on step gradient aqueous/organic wash cycles for (Grey circle) Phase A (this invention), (green triangle) Phase B (this invention), (blue square) conventional C18 packing under the same testing conditions. CAD detector, flow rate 1.0 ml/min, temperature 30 °C, (mobile phase A: 90:10 v/v% acetonitrile:0.1M ammonium acetate, pH 5.0; mobile phase B: 90:10 v/v% pure water:0.1M ammonium acetate, pH 5.0; Step gradient mob. Phase A0%-90%-0% over 200 min.

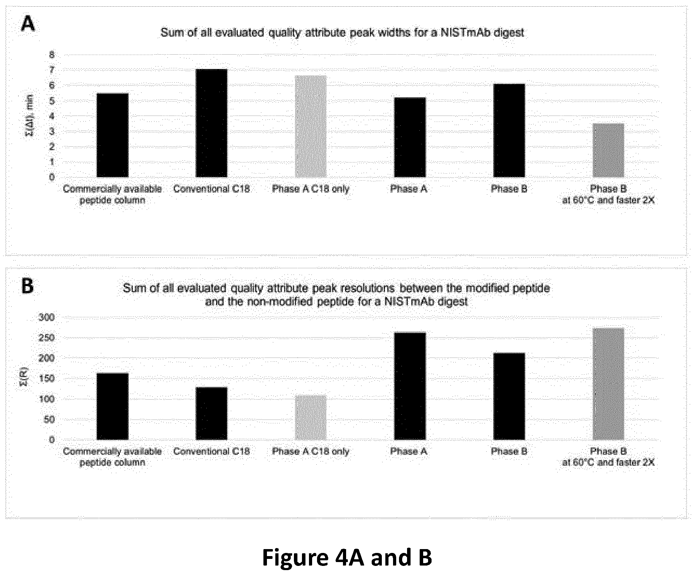

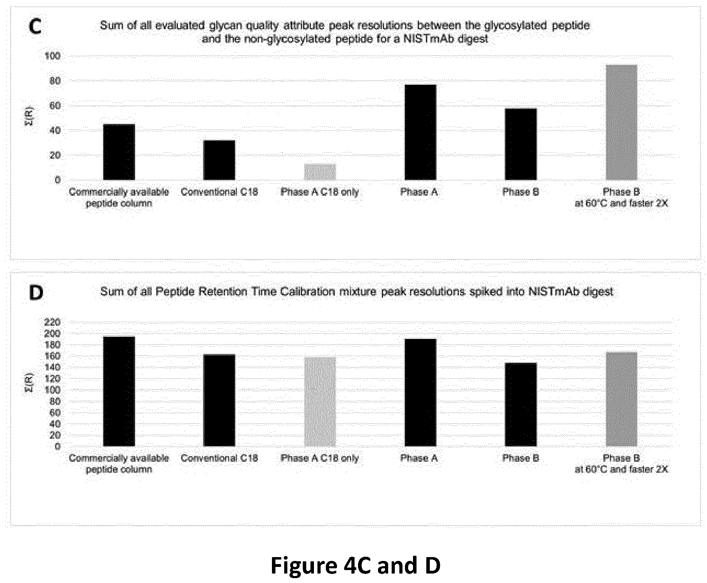

Figure 4: (A) Sum of all evaluated quality attribute peak widths for a NISTmAb digest analysed with LC-MS depending on the analytical column; (B) Sum of all evaluated quality attribute peak resolutions between modified peptide and the non-modified peptide for a NISTmAb digest analysed with LC-MS depending on the analytical column; (C) Sum of all evaluated glycan quality attribute peak resolutions between glycosylated peptide and the non-glycosylated peptide for a NISTmAb digest analysed with LC-MS depending on the analytical column; (D) Sum of all peptide retention time calibration mixture peak resolutions spiked into NISTmAb digest and analysed with LC-MS depending on the analytical column.

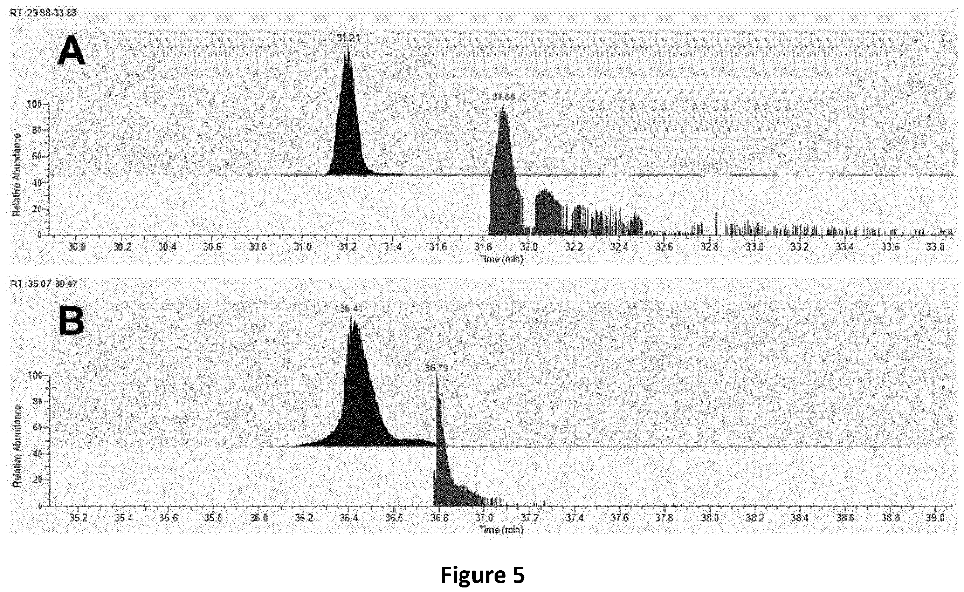

Figure 5: Separation of the "GFYPSDIAVEWESNGQPENNYK" peptide and its deaminated forms compared between Phase B (A) and conventional C18 packing (B).

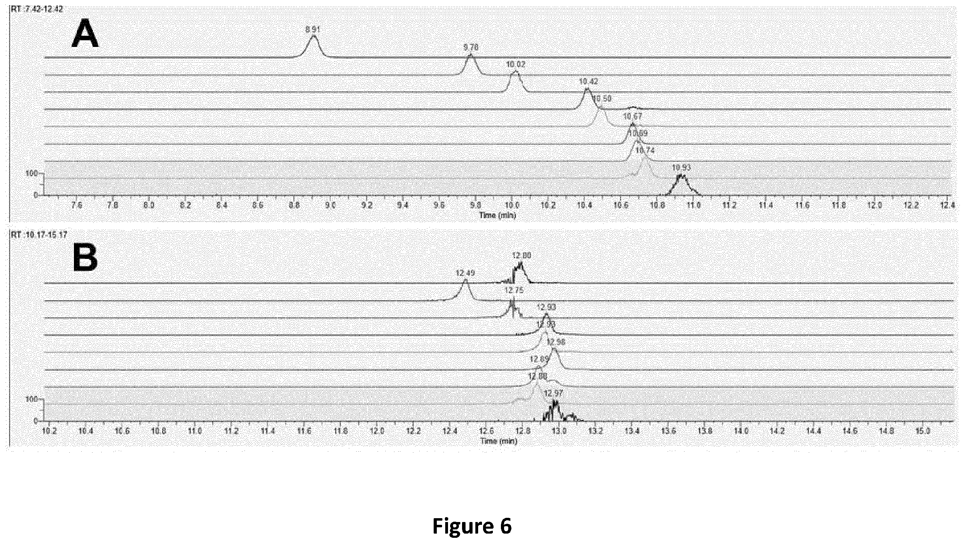

Figure 6: Separation of "EEQYNSTYR" peptide and its 8 glycosylated forms compared between Phase B (A) and conventional C18 packing (B).

In order to illustrate the present invention, the following non-limited examples of its practice are given below.

### Example 1 - Synthesis of the material for use as chromatography stationary phase material

### 1.1 Preparation of vinyl functionalized silica (Scheme 1) Step (a) (i):

20 g of dried solid-core porous spherical silica particles (dp, 2.0 µm; surface area, 75 m²/g; pore size, 160 Å) (Phase A), or fully porous spherical silica particles (dp, 1.9 µm; surface area, 170 m²/g; pore size, 150 Å) (Phase B), were transferred into a 250 - mL round bottom flask followed by the addition of a mixture of 7g of vinylethoxysiloxane homopolymer (e.g.: Gelest) and 0.44g of tetramethylethylenediamine (e.g.: Sigma-Aldrich) in toluene (60 mL). After carefully dispersing above slurry, the reaction mixture was put under stable refluxing under inert atmosphere and stirred for 48 h. The silica particles were filtered and thoroughly washed with acetone, acetone:water solution (1:1, v/v), acetonitrile:water solution (1:1, v/v), and followed by the mixture of 5 % formic acid and acetonitrile:water solution (1:1, v/v). After filtration and being washed with acetonitrile:water solution (1:1, v/v), acetone:water solution(1:1, v/v) and acetone, the resulting silica was dried under vacuum at 140 °C. for overnight. The dried silica was re-dissolved in 60 mL of toluene followed by the addition of 7 g of vinyldimethylethoxysilane (e.g.: Gelest) and 0.56 g of tetramethylethylenediamine (e.g.: Sigma-Aldrich). The resulting mixture was refluxed for 16 h. The functionalized silica particles were filtered and thoroughly washed with toluene, dioxane, methanol and acetone to give vinyl functionalized silica.

### 1.2 Preparation of polymer encapsulated silica phase using free radical polymerization (Scheme 2) Step (a) (ii):

12 mL of a dichloromethane was added to 10 g of vinyl functionalized silica (Phase A or Phase B), 2 g of 1 - octadecene (e.g.: Sigma-Aldrich), and 0.4 g of Dicumyl peroxide (e.g.: Sigma-Aldrich). The resulting mixture was sonicated until uniformity and then all volatiles were removed at reduced pressure with a rotary evaporator. Next, the resulting solvent-free mixture was transferred into the reactor, the reactor was sealed followed by flushing with an inert gas (e.g., nitrogen or argon) for 15 min, and heated to 160 ° C.

After being kept at the same temperature for 16 h, the reaction was cooled down, and the reaction mixture was dispersed in heptane and sonicated for 15 min. After filtration, the cake was thoroughly washed with toluene, dioxane, methanol and acetone to give polymer encapsulated silica Phase A or Phase B depending on the substrate particles used in 1.1.

### 1.3 Re-polymerization process (Scheme 3) Step (a) (iii):

The second polymerization cycle is performed to embody amine fragments to polymer encapsulated silica Phase A or Phase B as well as to cross-link the previously added polymer layer.

12 mL of a dichloromethane was added to 10 g of polymer encapsulated silica and 0.2 g of Dicumyl peroxide (e.g.: Sigma-Aldrich). The resulting mixture was sonicated until uniformity and then all volatiles were removed at reduced pressure with a rotary evaporator.

Next, the resulting mixture was transferred into the reactor and followed by flushing with an inert gas (e.g., nitrogen or argon) for 15 min. Next, 62.4 µL of N, N - Diisopropylethylamine (e.g.: Sigma-Aldrich) was added to the mixture, and the reactor was sealed and heated to 160 ° C. After being kept at the same temperature for 16 h, the reaction was cooled down, and the reaction mixture was dispersed in heptane and sonicated for 15 min. After filtration, the cake was thoroughly washed with toluene, dioxane, methanol and acetone to give mixed-mode phase stationary Phase A and Phase B.

This novel chromatographic stationary phase has two chemistries within a single column, allowing improved analysis of post-translational modifications and other impurities. Compared to conventional reversed-phase chromatography, mixed-mode columns provide a broader selectivity range, giving the user alternatives to improve the resolution of the separation.

### Example 2 - Application of Stationary Phase as Prepared in Example 1

The Mixed-mode stationary phase as described in Example 1 was packed into a chromatographic column (150mm×2.1mm I.D.) and applied for separation of the negatively charged analyte (chloride ions) under different buffer concentrations. Figure 1 shows the influence of ammonium formate concentration (counter-ion) on retention of negatively charged analyte, while maintaining the ACN content and pH of the mobile phase constant, on the Phase A, Phase B and Conventional C18 columns. As expected, the retention for Conventional C18 column remain unaltered within the change in counter-ion concentration. The retention is affected when a mixed-mode retention mechanism is present (Phase A and Phase B). The retention factor of negatively charged analyte increased when the counter-ion buffer concentration increased indicating that this invention synthetic approach delivers a mixed-mode phase with ion-pairing property.
Column: 150 mmx2.1 mm I.D.
Mobile Phase: 100mM ammonium formate pH 3.0, Acetonitrile, DI water
Flow rate: 0.3 mL/min
Column temperature: 30 °C
Detector: Charged Aerosol Detector (CAD)

### Example 3 - Application of stationary phase material A and B as Prepared in Example 1

### 2.1 Column Stability Testing

In order to demonstrate phase stability under stressful environment, hydrolytic stability and CAD detection background noise tests were applied for the columns.

Hydrolytic stability test was carried out on column (4.6x50mm) packed with Phase B using a mobile phase containing 0.1% TFA (aqueous) at flow rate 0.4 mL/min and at temperature of 50 °C. At 4 h intervals, the performance test was applied with sample containing acenaphthene (uracil as a void volume marker) and mobile phase containing ACN:0.01M ammonium acetate pH 5.0 (1:1, v/v) at 0.4 ml/min and 50 °C. The performance tests and low pH mobile phase treatment were repeated for 11 times (40 h in total).

A decrease in the retention factor is caused by hydrolysis of the bonded groups, with the hydrolysis products being removed by the mobile phase during the performance experiment.

Representative result of performance tests from Phase B column are compared to conventional C18 (Figure 2), there particles have equivalent size and surface parameters. The rate of retention decrease for acenaphthene on conventional C18 column is much higher than Phase B column, there 5.7% and 0.8% decrease was observed after 40 h, respectively. This demonstrates that Phase B is highly stable under acidic condition compared to the conventional C18, and that this feature is certainly beneficial for LC-MS application.

In order to estimate the possible column bleed, the Charged Aerosol Detection (CAD) is used (Figure 3). In CAD analysis, the eluent is nebulized with nitrogen and the droplets are dried to remove mobile phase, producing non-volatile analyte particles.

The signal intensity generated by a CAD is directly proportional to the analyte concentration. This means that the detector response for the bleed of a given column should be correlated to the amount of particulate matter per unit of time. The Phase A, Phase B and Conventional C18 columns were analysed with CAD (Figure 3), where the baseline signal was monitored under different aqueous and organic conditions. Conventional C18 column exhibits the highest intensity of background signal, indicating continuous bleed of the column.

In contrast to this, column prototypes with Phase A and Phase B of this invention gives minimal signal intensity, demonstrating excellent phase stability under aqueous and ACN condition. This data is well aligned with Hydrolytic stability test results, indicating that proposed synthesis method delivers a highly stable phase without any column bleed.

### 2.2 Peptide Separation

Glycopeptide identification relies on the very often incomplete chromatographic separation on column and fragmentation of both the peptide and the glycan in a mass spectrometer, which is further complicated by precursor ion heterogeneity due to charge states and adducts, as well as unintended collisional dissociation which may yield isobaric but structurally distinct ions, thus making the accurate, reliable and sensitive coverage of all analyte ions within a single LCMS/MS acquisition difficult or impossible to achieve, leading to distorted, implausible or missing results. The key considerations for quantitation of peptides, including glycopeptides, are sensitivity, specificity, reproducibility, precision, and throughput. Specificity is especially important for glycopeptides because different glycopeptides have the same peptide backbone and different glycopeptides share very similar MS/MS spectra.

A column packed with this invention mixed-mode stationary Phase A or Phase B are suitable for separation of glycosylated, deamidated and oxidized peptides with excellent resolution of the multiple modified peptide peaks from the respective non-modified peptide peaks. The improved chromatographic separation of molecules, and of glycopeptides particularly, improves the resolution and thus the sensitivity and specificity of peptide and glycopeptide identification.

The packing material may be used in a chromatographic separation to separate and analyse modified and non-modified peptides, including glycopeptides, at column compartment temperatures 30-80°C with mobile phases containing water, acetonitrile, methanol, ammonia, acetic acid, formic acid, trifluoroacetic acid, trichloroacetic acid.

NISTmAb tryptic digest was analysed, and Phase A and Phase B columns results were compared to commercially available peptide column (130Å, 1.7 µm, 2.1 × 150 mm), and conventional C18 column (1.9 µm, 2.1 × 150 mm), and presented in Figure 4 A-D, Figure 5, and Figure 6.

NISTmAb standard antibody digest for evaluation of peptide separations was prepared as follows. 10 µL portion of the 10 mg/mL NISTmAb sample was diluted with 90 µL of 7 M guanidine hydrochloride and chemically reduced for 30 minutes at room temperature with 2 µL of 500 mM dithiothreitol. Alkylation was carried out for 20 minutes at room temperature in dark with 4 µL of 500 mM sodium iodoacetate, halted by addition of 4 µL of 50 mM dithiothreitol. Buffer exchange was performed using Bio-Spin^{®} P-6 gel columns (Bio-Rad Laboratories, CA, USA) using Tris buffer at pH 7.9, collecting the reduced and alkylated sample in a microcentrifuge tube. This solution was digested with 1 mg/mL trypsin (Pierce^{™} Thermo Scientific^{™}, MA, USA) in 1:10 ratio with the sample. The resulting solution was heated at 37 °C for 30 minutes. Digestion was halted by 10% formic acid in 1:10 ratio. To evaluate retention and peak shape of the Pierce^{™} Peptide Retention Time Calibration Mixture peptides (Pierce^{™} Thermo Scientific^{™}, MA, USA), 5 pmol/µL solution of it was added in 1:40 ratio, yielding 40 pmol of the NISTmAb to 1 pmol of the Peptide Retention Time Calibration in an 8 µL sample injection for evaluation of the stationary phase material packed in stainless steel column.

Analysis was performed with Vanquish Horizon chromatographer coupled to Orbitrap Exploris mass spectrometer operated in full scan (resolution set at 120'000) and targeted fragmentation mode. The data was processed with BioPharma Finder software and extracted chromatograms were integrated using Multi-Attribute Method (MAM) (Thermo Scientific^{™}, MA, USA) for peptide mapping and quantification of quality attributes.

In Figure 4 A, the sum of peak widths of the quality attribute peaks for the mixed-mode stationary phase columns are shown to have been comparable to the commercial columns, furthermore the total peak width was improved by the faster analysis with modified gradient and higher temperature.

In Figure 4 B, the sum of the resolutions of all modified peptide quality attribute peaks from their respective non-modified peptide peaks for the mixed-mode stationary phase column is shown to have been advantageous, furthermore the sum of resolutions was improved by the faster analysis with modified gradient and higher temperature.

In Figure 4 C, only the glycopeptide sum of the resolutions is considered, for that reason the mixed-mode stationary phase appears to have been even more advantageous than in Figure 4 B.

In Figure 4 D, the sum of resolutions of the Peptide Retention Time Calibration Mixture peaks for the mixed-mode stationary phase column is shown to have been comparable to the commercial columns, furthermore the total of resolutions was improved by the faster analysis with modified gradient and higher temperature.

The mixed-mode stationary phase column separated N-glycan peptide with greater selectivity and resolution than the other RP-LC columns (Figure 4A and B, and Figure 4C and D). These properties of the present invention enable the successful identification of modified peptides at increased detail compared to conventional columns in analysis of enzymatically digested samples of proteins including therapeutical antibodies. Phase A C18 only represents a quality control for a phase without ion-pairing feature. Phase B at 60°C and faster 2X' represents the Phase B accelerated run at double flow rate and half analysis duration with column compartment temperature increased to 60°C. The 1X analysis conditions included a gradient of 1-2%B in 10 min followed by 2-35%B in 70 min at 0.250 mL/min and 50°C column compartment temperature. The mobile phase A was 0.1% formic acid in water and mobile phase B was 0.1% formic acid in acetonitrile.

Figure 5 shows the separation for the deamidated peptide "GFYPSDIAVEWESNGQPENNYK" and its deaminated forms compared between Phase B (Figure 5, A) and conventional C18 packing (Figure 5, B). The difference of retention time between first modified peptide peak and non-modified peptide peak is increased with Phase B column, and significantly improved isomer separation was observed.

Figure 6 shows the separation of different glycoforms of "EEQYNSTYR" peptide and its 8 glycosylated forms compared between Phase B (Figure 6, A) and conventional C18 packing (Figure 6, B). The resolution of peaks was increased with the Phase B column, and peak profile signal-to-noise was significantly improved, possibly due to reduction of co-elution.

Conventional reverse phase columns typically separate the glycopeptides based on the peptide portion, most of the glycoforms containing the same peptide sequence co-elute. This co-elution is disadvantageous in that abundant glycoforms can suppress the signals of other species. Mixed-mode columns provide an alternative separation strategy in that the glycoforms interact through ion exchange and reversed phase mechanism in the column; thus, the glycoforms for a given peptide are more readily separated (Figure 6, A).

The stationary phase of the present invention exhibits excellent low pH stability, with no detectable column bleeding, making it a highly desirable option for LC/MS applications. Its versatility is demonstrated by its successful application for modified peptide, including glycopeptide, detection.

## Claims

1. A method for making chromatographic packing material, wherein the packing material comprises a functionalised silicone-based polymer layer bonded to substrate particles and the method for forming the packing material comprises:
(a)
(i) forming silicone-based polymer encapsulated substrate particles comprising pendant functional groups by reacting functional groups on the substrate particles with a silicone-based polymer product comprising pendant functional groups;
(ii) functionalising the silicone-based polymer encapsulated substrate particles by reacting the pendant functional groups on the silicone-based polymer encapsulated substrate particles with at least one hydrophobic compound to form first functionalised silicone-based polymer encapsulated substrate particles; and
(iii) functionalising the first functionalised silicone-based polymer encapsulated substrate particles by reacting the first functionalised silicone-based polymer encapsulated substrate particles with at least one amine containing compound to form second functionalised silicone-based polymer encapsulated substrate particles; or
(b)
(i) forming a first functionalised silicone-based polymer by reacting pendant functional groups on a silicone-based polymer product with at least one hydrophobic compound;
(ii) functionalising the first functionalised silicone-based polymer by reacting the first functionalised silicone-based polymer with at least one amine containing compound to form second functionalised silicone-based polymer; and
(iii) reacting the second functionalised silicone-based polymer (product of step b (ii)) with functional groups on the substrate particles.

2. The method according to claim 1, wherein the functional groups on the substrate particles are selected from hydroxyl, epoxy and thiol.

3. The method according to claim 1 or 2, wherein the substrate particles are selected from the group consisting of metal oxides and organic polymers.

4. The method according to any one of the preceding claims, wherein the pendant functional groups on the silicone-based polymer encapsulated substrate particles or the silicone-based polymer product comprise groups capable of participating in a radical polymerisation reaction.

5. The method according to any one of the preceding claims, wherein the pendant functional groups on the silicone-based polymer encapsulated substrate particles or the silicone-based polymer product are reactive olefinic groups or reactive thiol groups, preferably olefinic groups.

6. The method according to claim 5, wherein the reactive olefinic groups or reactive thiol groups are selected from vinyl, allyl, methacrylate, acrylate, acrylamide, methacrylamide and styrene.

7. The method according to any one of the preceding claims, wherein the silicone-based polymer product comprises at least one leaving group.

8. A method according to claim 7, wherein the at least one leaving group is an alkoxy group.

9. The method according to any one of the preceding claims, wherein the silicone-based polymer product comprises vinylsiloxane.

10. The method according to claim 9, wherein the vinylsiloxane polymer has the formula 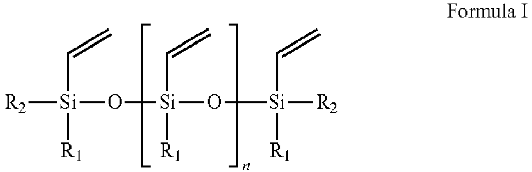 wherein n is an integer from 3 to 100, R₁ and R₂ are independently selected from the group consisting of: alkoxy, hydroxyl and halo.

11. The method according to claim 10, wherein R₁ and R₂ are independently selected from the group consisting of: methoxy, ethoxy and hydroxyl.

12. The method according to claim 10 or 11, wherein the vinylsiloxane polymer is a co-polymer.

13. The method according to any one of the preceding claims, wherein the at least one hydrophobic compound is a C₄ to C₃₀ alkyl.

14. The method according to claim 13, wherein the C₄ to C₃₀ alkyl is monounsaturated.

15. The method according to any one of the preceding claims, wherein the at least one amine containing compound is a polymerizable amine containing compound.

16. The method according to claim 15, wherein the amine in the at least one polymerizable amine containing compound is a tertiary amine.

17. The method according to any one of the preceding claims, wherein the reaction between the pendant functional groups on the silicone-based polymer encapsulated substrate particles in step (a) (ii) and the at least one hydrophobic compound is a free-radical polymerisation and forms an additional polymer layer bonded to the silicone-based polymer encapsulated substrate particles.

18. A method according to any one of the preceding claims, wherein the reaction between the first functionalised silicone-based polymer encapsulated substrate particles and at least one amine containing compound in step (a) (iii) is a free-radical polymerisation and forms an additional polymer layer bonded to the first functionalised silicone-based polymer encapsulated substrate particles.

19. A method according to any one of the preceding claims, wherein the reaction between the first functionalised silicone-based polymer encapsulated substrate particles and at least one amine containing compound in step (a) (iii) is a free-radical polymerisation and forms crosslinks in the first functionalised silicone-based polymer encapsulated particles.

20. The method according to any one of claims 1 to 16, wherein the reaction between the pendant functional groups on the silicone-based polymer product in step (b) (i) and the at least one hydrophobic compound is a free-radical polymerisation and forms an additional polymer layer bonded to the silicone-based polymer product.

21. The method according to any one of claims 1 to 16 and 20, wherein the reaction between the first functionalised silicone-based polymer and at least one amine containing compound in step (b) (ii) is a free-radical polymerisation and forms an additional polymer layer bonded to the first functionalised silicone-based polymer.

22. The method according to any one of claims 1 to 16, 20 and 21, wherein the reaction between the first functionalised silicone-based polymer and at least one amine containing compound in step (b) (ii) is a free-radical polymerisation and forms crosslinks in the first functionalised silicone-based polymer.

23. A method according to any one of the preceding claims, wherein the ratio of silicone-based polymer encapsulated substrate particles and the hydrophobic compound and/or the ratio of first functionalised silicone-based polymer encapsulated substates particles and the at least one amine containing compound compound may be from about 1:1 to about 200:1,.

24. The method of any one of the preceding claims wherein the packing material is provided in a form suitable for use as chromatographic packing.

25. A chromatographic packing material formed by the method of any one of the preceding claims.

26. A chromatographic packing material comprising:
(i) Substrate particles;
(ii) A functionalised silicone-based polymer bonded to the substrate particles prepared by:
(a)
(i) forming silicone-based polymer encapsulated substrate particles comprising pendant functional groups by reacting functional groups on the substrate particles with a silicone-based polymer product comprising pendant functional groups;
(ii) functionalising the silicone-based polymer encapsulated substrate particles by reacting the pendant functional groups on the silicone-based polymer encapsulated substrate particles with at least one hydrophobic compound to form first functionalised silicone-based polymer encapsulated substrate particles; and
(iii) functionalising the first functionalised silicone-based polymer encapsulated substrate particles by reacting the first functionalised silicone-based polymer encapsulated substrate particles with at least one amine containing compound to form second functionalised silicone-based polymer encapsulated substrate particles; or
(b)
(i) forming a first functionalised silicone-based polymer by reacting pendant functional groups on a silicone-based polymer product with at least one hydrophobic compound;
(ii) functionalising the first functionalised silicone-based polymer by reacting the first functionalised silicone-based polymer with at least one amine containing compound to form second functionalised silicone-based polymer; and
(iii) reacting the second functionalised silicone-based polymer (product of step b (ii)) with functional groups on the substrate particles.

27. The packing material according to any one of claims 26, wherein the packing is obtained using a method as defined in any one of claims 1 to 24.

28. The use of packing material according to claim 26 or 27 in chromatographic separation.

29. The use according to claim 28, wherein the chromatographic separation comprises the separation of glycopeptides,

30. The use according to claim 28 or 29, wherein the chromatographic separation is conducted using a mobile phase with a pH 1.0 or less.

31. The use according to claim 28 or 29, wherein the chromatographic separation is conducted using a mobile phase with a pH 13.0 or more.
